# EUROPEAN PATENT APPLICATION

(11) **EP 4 454 615 A1**
(43) Date of publication of application: **30.10.2024**
(21) Application number: 24172684.3
(22) Date of filing: 26.04.2024
(51) Int. Cl.: A61F 2/30, A61F 2/38

(54) **TOTAL KNEE REVISION ARTHROPLASTY IMPLANT KIT**

(30) Priority: 28.04.2023 US 202363462632 P
(71) Applicant: Howmedica Osteonics Corp., Mahwah, NJ 07430 (US)
(72) Inventor: COLLAZO, Carlos E., Old Greenwich, 06870 (US)
(74) Representative: Eisenführ Speiser

(57) **Abstract**

In one embodiment, the present disclosure relates to a joint replacement assembly (100a, 100b, 100c, 100d, 100e). The joint replacement assembly comprises a baseplate (110) having an articulating side and a bone facing side, and a proximal member (120, 520) having a proximal end (120a, 520a) and a distal end (120c, 520c), the proximal end being connectable to the bone facing side of the baseplate, the distal end having a plurality of grooves (121, 521) arranged about a first axis (A1) extending through the proximal member in a lengthwise direction. The joint replacement assembly further comprises a distal member (130, 230, 330, 430) having a proximal end (130a, 230a, 330a, 430a) and a distal end (130c, 230c, 330c, 430c), the proximal end being connectable to the distal end of the proximal member and having a plurality of protrusions (131, 231, 331, 431) configured to engage with the plurality of grooves and prevent rotation of the distal member about the first axis relative to the proximal member.

## Description

### CROSS-REFERENCE TO RELATED APPLICATIONS

This application claims the benefit of the filing date of United States Provisional Patent Application No. 63/462,632 filed April 28, 2023, the disclosure of which is hereby incorporated herein by reference.

### BACKGROUND

Revision knee replacement surgery is oftentimes a challenging and somewhat unpredictable procedure depending on the severity of the condition being treated for a particular patient. The removal of a primary total knee may result in moderate to severe bone loss, as well as ligamentous structures being compromised. Such bone loss varies greatly in size, shape, and location. Bone loss also varies in its quality which ranges from adequately dense and appropriate for biologic fixation, to soft and osteopenic bone better suited for cement fixation. Bone loss may be generally planar across a portion of a patient's tibial plateau, and other times bone loss may span the entire width of the tibia. Further, the bone loss may be a cavitary defect, such as a truncated void into the metaphyseal region of the tibia. The location of the cavitary defect may be close or more distant in proximity to the intramedullary ("IM") canal resulting in fixation challenges since the IM canal is almost always used for fixation purposes as it provides a stable anchoring means when used with an intramedullary stem. Furthermore, the bone loss may be a combination of both planar and cavitary defects.

The clinical success and longevity of a knee revision depends primarily in the overall fixation and stability of a revision knee implant. Manufacturers of such implants offer various options to address the challenges listed above for revision knee replacement surgery. Metaphyseal void fillers and flat augments are offered to address cavitary defects, and it is not uncommon to use a metaphyseal void filler in conjunction with a stem offset adapter. Depending on the implant system being used, an offset adapter is either placed distal to the void filler or within the void filler. Placing the offset adapter distal to the void filler may have size restrictions since the offset adapter may be at a depth in the tibia where the metaphyseal region "necks" down in size as it transitions from a flared metaphyseal region to a proximal tibial shaft region. A larger offset, for example, may not fit or may present the risk of perforating the cortical wall of the bone during the reaming preparation for an implant. On the other hand, an offset adapter placed within a hollow void filler may result in a similar size restricting scenario wherein the amount of offset required may be limited by the internal geometry of the void filler. Currently, these types of hollow void fillers have to be set deeper into a tibia to provide additional clearance for a stem offset adapter, which may require more bone to be removed to accommodate a deeper void filler.

Another drawback of using certain metaphyseal styles of void fillers, for example those void fillers that are implanted into the tibia before the implantation of the baseplate and stem assembly, is that the diameter of the stem is limited by the size of the internal bore on the void filler. For example, if the size of a defect dictates using a smaller void filler and the surgeon desires to press fit an intermedullary stem into a large intramedullary canal, the size of the stem may exceed the opening in the void filler by which the stem is passed through during implantation.

Yet another drawback is presented by metaphyseal void fillers, stem offset adapters and augments that are mechanically or adhesively fixed to a distal surface of the baseplate. There are instances when a knee revision component itself has to be revised. One reason could be infection in the joint. In such scenario, the implant may be well fixed by either cement fixation, bone ingrowth or a combination of both. There may be nothing wrong with the fixation of the implant other than it has to be removed for direct access for the treatment with antibiotics. Since there is no access to easily decouple the components implant, removal of the entire assembly is typically required. Such removal is both tedious and time consuming, inevitably resulting in undesired boss loss in the process. Thus, further improvements are desirable.

### BRIEF SUMMARY

In a first example of a first aspect, the present disclosure relates to a joint replacement assembly. The joint replacement assembly comprises a baseplate having an articulating side and a bone facing side. The joint replacement assembly further comprises a proximal member having a proximal end and a distal end, the proximal end being connectable to the bone facing side of the baseplate, the distal end having a plurality of grooves arranged about a first axis extending through the proximal member in a lengthwise direction. The joint replacement assembly further comprises a distal member having a proximal end and a distal end, the proximal end being connectable to the distal end of the proximal member and having a plurality of protrusions configured to engage with the plurality of grooves and prevent rotation of the distal member about the first axis relative to the proximal member.

In a second example, the first example of the first aspect is further defined wherein the plurality of grooves are arranged in 30 degree increments about the first axis. In a third example, the first example of the first aspect is further defined wherein the distal end of the distal member includes a distal bore configured to engage an intramedullary stem. In a fourth example, the third example of the first aspect is further defined wherein the distal bore is coaxial with the first axis of the proximal member. In a fifth example, the third example of the first aspect is further defined wherein the distal bore is centered about an axis laterally offset from the central axis of the proximal member. In a sixth example, any one of the third, fourth and fifth examples of the first aspect are further defined wherein the proximal member includes a proximal bore coaxial with the first axis.

In a seventh example, the first example of the first aspect is further defined wherein the baseplate, proximal member, and distal member are each formed separately from each other. In an eight example, the first example of the first aspect is further defined wherein a distal surface of the baseplate includes a hole extending through the baseplate. In a ninth example, the eight example of the first aspect is further defined wherein a proximal surface of the proximal member includes a first protrusion configured to engage with the hole of the baseplate to prevent rotation of the proximal member relative to the baseplate.

In a tenth example, the first example of the first aspect is further defined wherein the distal member includes a proximally extending boss and the baseplate includes a distally extending boss, the proximally extending boss being extendable through the proximal member and receivable within the distally extending boss of the baseplate. In an eleventh example, the tenth example of the first aspect is further defined wherein the proximally extending boss of the distal member is threaded such that a fastener extending through the baseplate and engaging the proximally extending boss secures the baseplate, proximal member, and distal member together.

In a twelfth example, the first example of the first aspect is further defined wherein the proximal member is a proximal void filler having a frustoconical body having an outer surface tapering inwardly from the proximal end to the distal end thereof. In a thirteenth example, the first example of the first aspect is further defined wherein the distal member is a distal void filler having a frustoconical body having an outer surface tapering inwardly from the proximal end to the distal end thereof. In a fourteenth example, the thirteenth example of the first aspect is further defined wherein the distal member further includes a lobe portion integral with the frustoconical body, the lobe portion extending along at least a portion of a length of the frustoconical body and having an axial bore extending into the lobe, the axial bore defining a second axis offset from the first axis by an offset distance. In a fifteenth example, the fourteenth example of the first aspect is further defined wherein the lobe portion and second axis are positionable at a plurality of rotational positions about the first axis.

In a first example of a second aspect, the present disclosure relates to a total knee revision arthroplasty implant kit. The total knee revision arthroplasty implant kit comprises a baseplate having a proximal surface for engaging tibial insert, a distal surface for engaging a bone, a first boss extending from the distal surface in a distal direction and defining a first bore, a second and third boss extending perpendicularly from the distal surface in the distal direction, and a first hole extending from the distal surface towards a proximal surface of the baseplate. The total knee revision arthroplasty implant kit further comprises a proximal void filler having an outer surface, a fin extending outwardly from the outer surface, a fourth bore extending from a proximal surface to a distal surface of the proximal void filler, a first protrusion extending perpendicularly from the proximal surface in a proximal direction, and a second protrusion extending from the proximal surface in the proximal direction, wherein the proximal surface of the proximal void filler is connectable to the distal surface of the baseplate, the outer surface is configured to engage the bone, the fourth bore is connected to the first boss, and the first protrusion is connected to the first hole. The total knee revision arthroplasty implant kit further comprises a distal void filler including an outer surface, a fourth boss extending from a proximal surface in a proximal direction, a fifth bore extending from a distal surface in a distal direction, wherein the proximal surface of the distal void filler is connectable to the distal surface of the proximal void filler, the outer surface is configured to engage the bone, the fourth boss is receivable within the first bore and the fifth bore is configured to receive an intramedullary stem. The total knee revision arthroplasty implant kit further comprises a first spacer portion including a first cutout extending from a proximal surface to a distal surface of the first spacer portion and an opening extending from the proximal surface to the distal surface, wherein the proximal surface of the first spacer is connectable to the distal surface of the baseplate, the distal surface of the first spacer is connectable to the proximal surface of the proximal void filler, the first cutout is connectable to the second protrusion, and the aperture is connectable to the second and third bosses. The total knee revision arthroplasty implant kit further comprises a second spacer portion including a second cutout extending from a proximal surface to a distal surface of the second spacer portion, wherein the proximal surface of the second spacer portion is connectable to the distal surface of the first spacer portion, the second cutout is connectable to the fin, and the distal surface is configured to engage the bone. The total knee revision arthroplasty implant kit further comprises a locking screw connectable to the first bore.

In a second example, the first example of the second aspect is further defined wherein the first boss includes a generally cylindrical outer surface. In a third example, the second example of the second aspect is further defined wherein the first bore is generally concentric with the outer surface of the first boss. In a fourth example, the third example of the second aspect is further defined wherein the first bore has a defined depth as to not protrude through the proximal surface of the baseplate.

In a fifth example, the first example of the second aspect is further defined wherein the first boss includes a cylindrical hole that is generally concentric to and smaller in diameter than the first bore and extends through the first bore to intersect the proximal and distal surfaces of the baseplate. In a sixth example, the first example of the second aspect is further defined wherein the second and third bosses each have smaller lengths and diameters than the first boss.

In a seventh example, the first example of the second aspect is further defined wherein the second and third bosses include generally cylindrical outer surfaces. In an eight example, the seventh example of the second aspect is further defined wherein the second and third bores are generally concentric with the outer surface of the second and third bosses. In a ninth example, the eighth example of the second aspect is further defined wherein the second and third bores have a depth as to not protrude the proximal surface.

In a tenth example, the first example of the second aspect is further defined wherein the first hole has a defined depth not to protrude through the proximal surface of the baseplate. In an eleventh example, the first example of the second aspect is further defined wherein the outer surface of the proximal void filler is tapered and adapted for cement or biological fixation. In a twelfth example, the first example of the second aspect is further defined wherein the fourth bore has a precision slidable fit with the first boss. In a thirteenth example, the first example of the second aspect is further defined wherein the first protrusion is generally cylindrical and has a precision slidable fit with the first hole. In a fourteenth example, the first example of the second aspect is further defined wherein the fin includes at least two fins.

In a fifteenth example, the first example of the second aspect is further defined wherein the distal surface of the proximal void filler includes groves longitudinally aligned perpendicular to an axis of the fourth bore. In a sixteenth example, the fifteenth example of the second aspect is further defined wherein twelve grooves are displaced about the axis of the fourth bore every 30 degrees. In a seventeenth example, the first example of the second aspect is further defined wherein the second protrusion includes a cylindrical outer surface extending circumferentially around the fourth bore. In an eighteenth example, the seventeenth example of the second aspect is further defined wherein the second protrusion has a precision locating fit with the first cutout. In a nineteenth example, the eighteenth example of the second aspect is further defined wherein the second protrusion has a planar edge at the proximal most portion of the second protrusion.

In a twentieth example, the first example of the second aspect is further defined wherein the outer surface of the distal void filler is tapered and adapted for cement or biological fixation. In a twenty first example, the first example of the second aspect is further defined wherein the fifth bore is threaded. In a twenty second example, the first example of the second aspect is further defined wherein the fourth boss is generally cylindrical and has a precision slidable fit with the first bore.

In a twenty third example, the first example of the second aspect is further defined wherein the proximal surface of the distal void filler includes groves longitudinally aligned perpendicular to an axis of the fourth boss. In a twenty fourth example, the twenty third example of the second aspect is further defined wherein twelve grooves are displaced about the axis of the fourth boss every 30 degrees.

In a twenty fifth example, the first example of the second aspect is further defined wherein the fifth bore is parallel and offset from an axis extending through the fourth boss. In a twenty sixth example, the twenty fifth example of the second aspect is further defined wherein the offset is 30 degrees.

In a twenty seventh example, the first example of the second aspect is further defined wherein the bone engaged by the distal portion of the second spacer portion is a tibial plateau. In a twenty eight example, the twenty seventh example of the second aspect is further defined wherein the first and second spacer portion span only a portion of the medial-lateral width of the tibia. In a twenty ninth example, the twenty seventh example of the second aspect is further defined wherein the first and second spacer portion span the entire medial-lateral width of the tibia.

In a first example of a third aspect, the present disclosure relates to a joint replacement system. The joint replacement system includes a baseplate having an articular side and a bone facing side. The joint replacement system further includes a proximal member having a first end and a second end, the first end being engageable with the bone facing side of the baseplate, and the second end having a first indexing feature. The joint replacement system further includes a distal member having a first end and a second end, the first end having a second indexing feature configured to engage and index with the first indexing feature so as to prevent relative rotation between the proximal and distal members when the distal member is engaged with the proximal member.

In a first example of a fourth aspect, the present disclosure relates to a joint replacement assembly. The joint replacement assembly includes a baseplate having an articular side and a bone facing side. The joint replacement assembly further includes a proximal member having a first end, a second end, and a first axis extending through the first and second ends, the first end removably engaged to the bone facing side of the baseplate. The joint replacement assembly further includes a distal member having a first end and a second end, the first end removably engaged to the second end of the proximal member, the second end having an opening defining a second axis, the second axis being offset from the first axis in a radial direction.

In a second example, the first example of the fourth aspect is further defined wherein the distal member is a distal void filler having a frustoconical body and a lobe portion connected to the frustoconical body, the opening extending into the lobe portion. In a third example, the second example of the fourth aspect is further defined wherein the lobe portion extends along at least a portion of a length of the frustoconical body. In a fourth example, the first example of the fourth aspect is further defined wherein the distal member has an axial opening extending therein and defining a second axis radially offset from the first axis.

In a first example of a fifth aspect, the present disclosure relates to a method of preparing a tibia to receive a joint replacement assembly. The method comprises connecting a tibial baseplate trial to a stem extending along a first axis and within an intramedullary canal of the tibia; determining whether an offset is required by assessing the coverage of a tibial plateau of the tibia with the tibial baseplate trial; if an offset is required: connecting the tibial baseplate trial to the stem via an offset stem adapter such that a boss of the tibial baseplate trial extends along the first axis and the stem extends along a second axis and within the intramedullary canal of the tibia, rotating the tibial baseplate trial, offset stem adapter, and stem from a first rotational orientation to a second rotational orientation, marking the tibia to record the second rotational orientation, reaming the tibia along the first axis, aligning a cutting guide with the marking on the tibia, and resecting the tibial plateau with the cutting guide.

### BRIEF DESCRIPTION OF THE DRAWINGS

The features, aspects, and advantages of the present disclosure will become better understood with regard to the following description, appended claims, and accompanying drawings in which:
FIG. 1A is a perspective view of a joint replacement assembly according to an embodiment of the present disclosure.
FIG. 1B is another perspective view of the joint replacement assembly according to FIG. 1A.
FIG. 1C is a cross-sectional view of the joint replacement assembly of FIG. 1A taken along a midline thereof.
FIG. 1D is a top view of the joint replacement assembly of FIG. 1A.
FIG. 2A is a bottom perspective view of a baseplate of the joint replacement assembly of FIG. 1A.
FIG. 2B is a cross-sectional view of the baseplate of FIG. 2A taken along a midline thereof.
FIG. 3A is a top perspective view of a proximal void filler of the joint replacement assembly of FIG. 1A.
FIG. 3B is a bottom perspective view of the proximal void filler of FIG. 3A.
FIG. 4 is a side view of a distal void filler of the joint replacement assembly according to FIG. 1A.
FIG. 5A is a top perspective view of a spacer of the joint replacement assembly of FIG. 1A.
FIG. 5B is a bottom perspective view of the spacer of FIG. 5A.
FIG. 6A is a perspective view of a joint replacement assembly according to another embodiment of the present disclosure.
FIG. 6B is a cross-sectional view of an offset distal void filler of the joint replacement assembly of FIG. 6A.
FIG. 6C is a top perspective view of a spacer of the joint replacement assembly of FIG. 6A.
FIG. 6D is a side-by-side elevational view of the assembly of FIG. 6A and a prior art joint replacement assembly.
FIG. 6E is a perspective view of a prior art cone-type void filler.
FIG. 7A is a perspective view of a joint replacement assembly according to another embodiment of the present disclosure.
FIG. 7B is a perspective view of a stem adapter of the joint replacement assembly of FIG. 7A.
FIG. 8A is a perspective view of a joint replacement assembly according to another embodiment of the present disclosure.
FIG. 8B is a perspective view of an offset stem adapter of the joint replacement assembly of FIG. 8A.
FIG. 9A is a perspective view of a joint replacement assembly according to another embodiment of the present disclosure.
FIG. 9B is a perspective view of a keel of the joint replacement assembly of FIG. 9A.
FIG. 10 is a perspective view of a joint replacement assembly according to another embodiment of the present disclosure.
FIG. 11A is a bottom perspective view of a trial baseplate according to an embodiment of the present disclosure.
FIG. 11B is a top perspective view of the trial baseplate of FIG. 11A.
FIG. 12A is a perspective view of a neutral stem extender according to an embodiment of the present disclosure.
FIG. 12B is another perspective view of the neutral stem extender of FIG. 12A.
FIG. 13 is a perspective view of an offset stem extender according to an embodiment of the present disclosure.
FIG. 14 is a perspective view of a trial stem according to an embodiment of the present disclosure.
FIG. 15 is a perspective view of a keel punch according to an embodiment of the present disclosure.
FIG. 16 is an exploded view of a reamer assembly according to an embodiment of the present disclosure.
FIG. 17 is a perspective view of a cutting guide according to an embodiment of the present disclosure.
FIG. 18 is a flowchart illustrating methods of using the devices of FIGS. 1A-17.
FIGS. 19-33 illustrate the methods diagramed in FIG. 18.

### DETAILED DESCRIPTION

As used herein, the term "proximal," when used in connection with a surgical tool or device, or components of a device, refers to the end of the device closer to the user of the device when the device is being used as intended. On the other hand, the term "distal," when used in connection with a surgical tool or device, or components of a device, refers to the end of the device farther away from the user when the device is being used as intended. As used herein, the terms "substantially," "generally," "approximately," and "about" are intended to mean that slight deviations from absolute are included within the scope of the term so modified, such as deviations of up to 10% greater or lesser than absolute. All vertical directional terms, such as "up," "down," "above," "below," "vertical," or "height" used in the following description refer only to the orientation of features as depicted in the figure being described. Such directional terms are not intended to suggest that any features of the devices described herein must exist in any particular orientation when constructed.

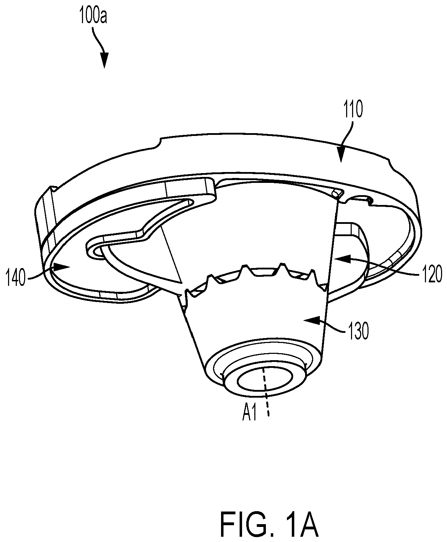

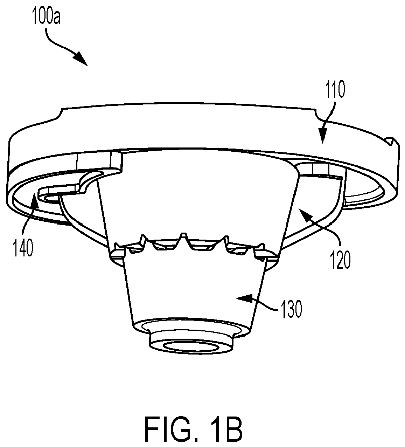

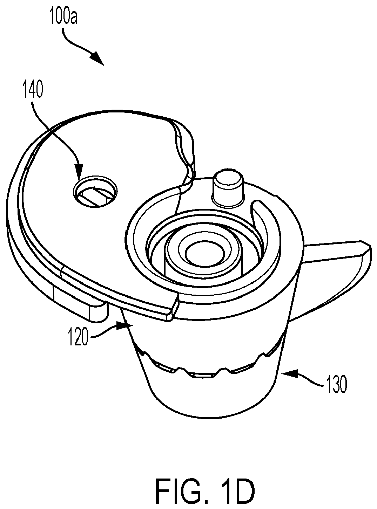

FIGs. 1A-5B depict a first joint replacement assembly 100a according to one embodiment of the present disclosure. The first joint replacement assembly 100a is particularly configured for addressing bone loss of a tibia during a revision total knee replacement surgery. The joint replacement assembly 100a includes a baseplate 110, a proximal void filler 120, a distal void filler 130, an augment spacer 140, and a locking screw 150.

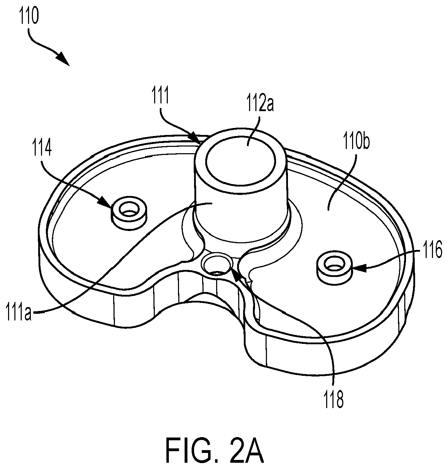

The baseplate 110 has a proximal side or articulating side and a distal side or bone facing side. The proximal side is defined at least partially by a proximal surface 110a and a distal surface 110b, as best shown in FIGs. 2A-2B. The proximal surface 110a is generally planar and includes a hole 113 that is generally cylindrical and disposed at or near a center of the proximal surface. The hole 113 extends perpendicularly along a first axis A1 relative to proximal surface 110a to an inner surface of a first boss 111, discussed in greater detail below. Further, the hole 113 is configured for engagement with the locking screw 150 when the joint replacement assembly 100a is in a fully assembled state. For example, hole 113 may be shaped to receive and conform to a head of locking screw 150. As shown, proximal surface 110a is recessed within baseplate 110 so as to form a tray configured to receive a tibial insert (not shown) for articulation with a femoral component (not shown), as is understood in the art.

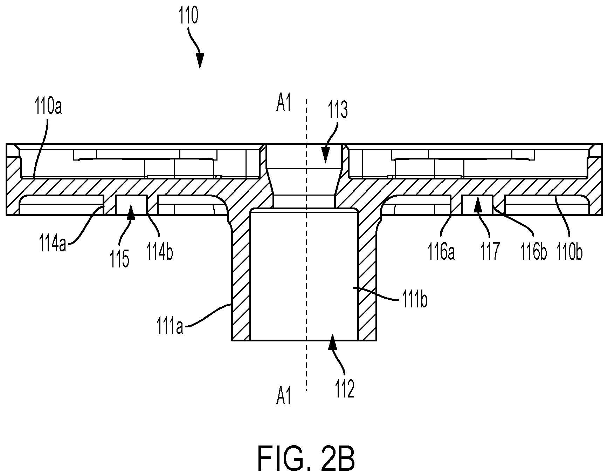

The distal side is at least partially defined by a distal surface 110b that is generally planar. A first boss 111, a second boss 114, and a third boss 116 extend perpendicularly from the distal surface 110b in a distal direction. The first boss 111 is disposed generally at or near a center point of the distal surface 110b. Second and third bosses 114, 116 are disposed to opposite sides (i.e., lateral and medial flanks) of the first boss 111 on the distal surface. The first, second, and third bosses 111, 114, 116 have generally cylindrical outer surfaces 111a, 114a, 116a, respectively, but are not limited to such shapes. Both an inner diameter and length of the first boss 111 is greater than the inner diameters and lengths of the second and third bosses 114, 116, respectively. The first boss 111 includes an inner surface 111b that is generally cylindrical in shape and defines an opening 112 that extends from the distal surface 110b to hole 113 of the proximal surface 110a of the baseplate 110. However, in some embodiments inner surface 111b may be conically tapered. In this regard, opening 112 is configured to receive a stem, stem adapter, metaphyseal cone, and the like, as discussed further below. Additionally, opening 112 is coaxial with hole 113 and coaxial with the first axis A1. The second and third bosses 114, 116 also include respective inner surfaces 114b, 116b that are generally cylindrical and define openings 115, 117, but the openings of the second and third bosses do not extend entirely through the baseplate 110 from the distal surface 110bto the proximal surface 110a. Such second and third bosses 114, 116 are generally configured to receive corresponding protrusions of other devices that can be coupled to baseplate 110, as described further below. The baseplate 110 further includes a hole 118 adjacent and posterior to the first boss 111. The hole 118 extends from the distal surface 110b to the proximal surface 110a and is generally cylindrical in shape. As best shown in FIGs. 2A and 2B, distal surface 110b is recessed within baseplate 110.

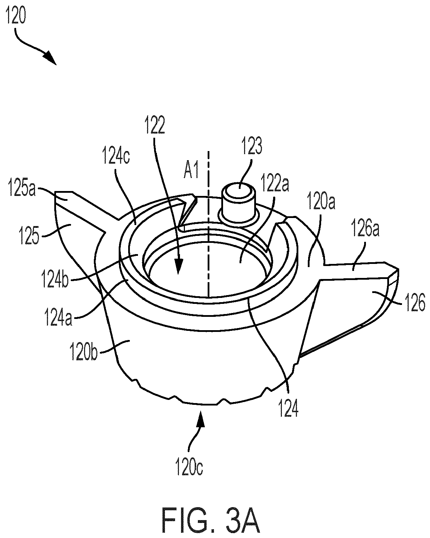

The proximal void filler or proximal member 120 is defined by a proximal surface 120a, an outer surface 120b, and a distal surface 120c, as best shown in FIGs. 3A-3B. The proximal surface 120a is generally planar and is defined along an outer circumference of a proximal opening of a bore 122. The bore 122 is generally cylindrical and extends perpendicularly from the proximal surface 120a to the distal surface 120c of the proximal void filler 120. The proximal surface 120a includes a first protrusion 123, and a second protrusion 124. The first protrusion 123 is generally cylindrical and extends proximally from proximal surface 120a on one side of the bore 122. The second protrusion 124 also extends proximally from proximal surface 120a and extends partially about a circumference or edge of the bore 122. Second protrusion 124 includes a cylindrical outer surface 124a and an inner surface 124b that conically tapers inwards toward bore 122a. The outer and inner surfaces 124a, 124b converge to a generally planar proximal surface 124c which defines a terminal end of protrusion 124.

The outer surface 120b of the proximal void filler 120 is generally frustoconical and tapers from the proximal surface 120a to the distal surface 120c. The outer surface 120b includes a first fin or keel 125 and a second fin or keel 126 extending in an outward direction. The fins 125, 126 each include a proximal surface 125a, 126a, respectively, that is generally planar and aligned with a radial extension of the proximal surface 120a of the proximal void filler 120. A distal surface 125b, 126b of each fin 125 and 126, respectively, is generally planar and is angled to taper down towards the distal surface 120c of the proximal void filler 120. Outer surface 120b may also be comprised of a porous structure configured for bony ingrowth.

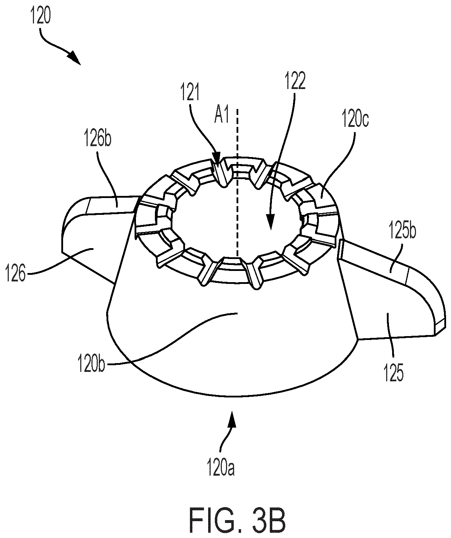

The distal surface 120c of the proximal void filler 120 extends about and defines a circumference of a distal opening of the bore 122, as best shown in FIG. 3B. The distal surface 120c has a smaller outer diameter than the proximal surface 120a due to the tapering of the proximal void filler 120. The proximal void filler 120 includes a plurality of notches or grooves 121 disposed upon the distal surface 120c. Such grooves 121 form a first indexing feature. As shown in FIG. 3B, the distal surface 120c includes twelve grooves 121 disposed equally along a central axis (i.e., axis A1) of the bore 122. For example, each groove 121 may be circumferentially offset from an adjacent groove 121 in increments of 10 to 60 degrees. In a preferred embodiment, as shown in FIG 3B, the increment is 30 degrees. In this regard, the distal surface 120c is not limited in the number of grooves 121 and, therefore, may include more than twelve grooves 121 or less than twelve grooves 121 depending on the incremental separation of grooves 121. It is also contemplated that the circumferential separation between some adjacent grooves 121 may be larger than between others.

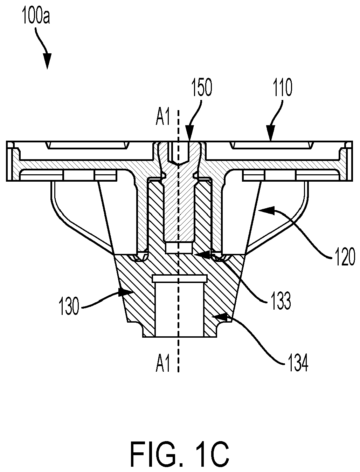

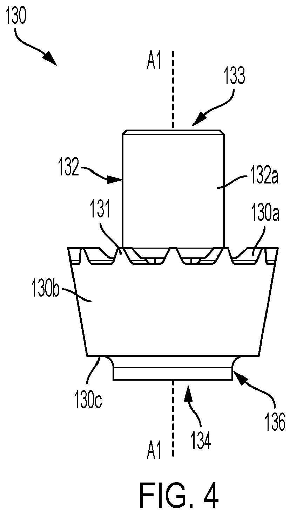

The distal void filler or distal member 130 is a neutral void filler that is defined by a proximal surface 130a, an outer surface 130b, and a distal surface 130c, as best shown in FIG. 4. The proximal surface 130a is defined along an outer circumference of a proximal boss 132 that extends perpendicularly from a proximal end of distal void filler 130 in a proximal direction and along first axis A1. The proximal surface 130a includes a plurality of protrusions or teeth 131 corresponding to grooves 121 of proximal void filler 130 and configured to mesh or interdigitate within grooves 121 which prohibits relative rotation and helps set a desired rotational alignment between proximal void filler 120 and distal void filler 130. In other words, protrusions 131 form a second indexing feature which index with the first indexing feature to establish a rotational relationship between proximal void filler 120 and distal void filler 130. It should be understood that in some embodiments, proximal void filler 120 may have protrusions while distal void filler may have grooves. Void filler 130 includes the same number of protrusions 131 as grooves 121 and such protrusions 131 are arranged around first axis A1 at the same circumferential intervals as that of grooves 121. Thus, in the embodiment depicted, void filler 130 includes twelve protrusions 131 disposed equally about a central axis A1 of the boss 132, i.e., every 30 degrees. However, as mentioned above with respect to proximal void filler 120, the proximal surface 130a is not limited to this configuration and may include more than twelve protrusions 131 or less than twelve protrusions 131 provided there is correspondence with grooves 121. The boss 132 has a generally cylindrical outer surface 132a, but, in some embodiments, outer surface 132a may be conically tapered so as to form a taper lock with bore 112a of baseplate 110. Boss 132 also includes a generally cylindrical inner surface 132b that is threaded to define a threaded bore 133 for threaded engagement with fastener 150. The threaded bore 133 has a defined depth that does not extend entirely through the distal void filler 130 to the distal surface 130c. In other words, it is a blind bore, as shown in FIG. 1C.

The outer surface 130b of the distal void filler 130 is generally frustoconical and tapers from the proximal surface 130a to the distal surface 130c. The distal surface 130c of the distal void filler 130 extends radially toward a distal boss 136 that extends distally therefrom. A distal bore 134 extends into a distal end of distal void filler 130 through distal boss 136. Such bore 134 is coaxial with threaded bore 133 and, therefore, is coaxial with first axis A1, as shown in FIG. 1C. Bore 134 may be a smooth bore or threaded bore and may be cylindrical or tapered.

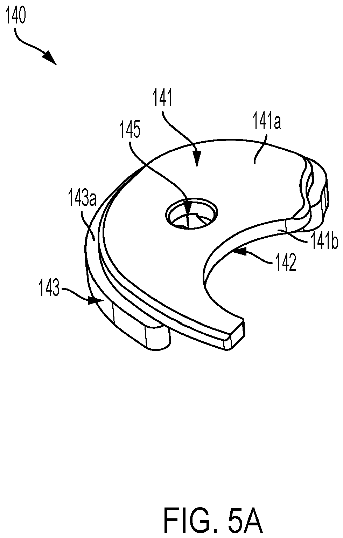

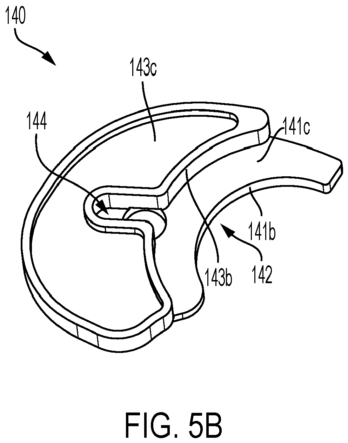

The augment spacer 140 includes a first spacer portion 141 and a second spacer portion 143, as best shown in FIGs. 5A-5B. The first spacer portion 141 is defined by a proximal surface 141a, a distal surface 141c, and a wall surface 141b disposed between the proximal surface 141a and the distal surface 141c. The proximal surface 141a and distal surface 141c are generally planar and include a hole 145 that is generally cylindrical and extends from the proximal surface 141a to the distal surface 141c at a center point of the first spacer portion 141. The wall surface 141b is generally curved and defines a cutout 142.

The second spacer portion 143 is defined by a proximal surface 143a, a wall surface 143b, and a distal surface 143c. The proximal surface 143a and the distal surface 143c are generally planar, and the proximal surface 143a is connected to or integral with the distal surface 141c of the first spacer portion 141. The second spacer portion 143 is laterally offset from a central point of the first spacer portion 141 such that a small portion of the proximal portion 143a of the second spacer portion 143 is exposed. The wall surface 143b is generally curved and defines an elongated cutout 144 disposed at a substantially center point of the second spacer portion 143. The cutout 144 is generally an elongate slot that is configured to expose hole 145 through cutout 144 and to receive a portion of either fin 125 or 126.

When the first joint replacement assembly 100a is fully assembled, as shown in FIGs. 1A-1C, the baseplate 110 is connected to the proximal void filler 120, the distal void filler 130, and the augment spacer 140. Specifically, the distal side of the baseplate 110 may be coupled to both the proximal end of the proximal void filler 120 and the proximal end of the first spacer portion 141. In this regard, outer surface 111a of the boss 111 of the baseplate 110 is received within bore 122 of the proximal void filler 120, as shown in FIG. 1C. Further, the first protrusion 123 of the proximal void filler 120 is received within hole 118 of the baseplate 110. The first cutout 142 and second cutouts 144 of the augment spacer 140 are configured to provide a precision coupling fit with proximal void filler 120 such that first spacer portion 141 extends partially about and generally conforms to the exterior of proximal void filler 120 and either fin 125 or 126 is received within second cutout 144. The augment spacer 140 spans only a portion of the medial-lateral width of the tibial plateau and, as such, may be used to augment a lateral or medial defect of a tibia. Therefore, when implanted, the proximal surface 141a of the first spacer portion 141 may rest flush against distal surface 110b of baseplate 110, while distal surface 143b of the second spacer portion 143 may rest flush against a resected surface of a tibial plateau for either cement fixation or biological ingrowth. The outer surface 120b of the proximal void filler 120 may be press-fit within a bone void of a tibia or cemented therein.

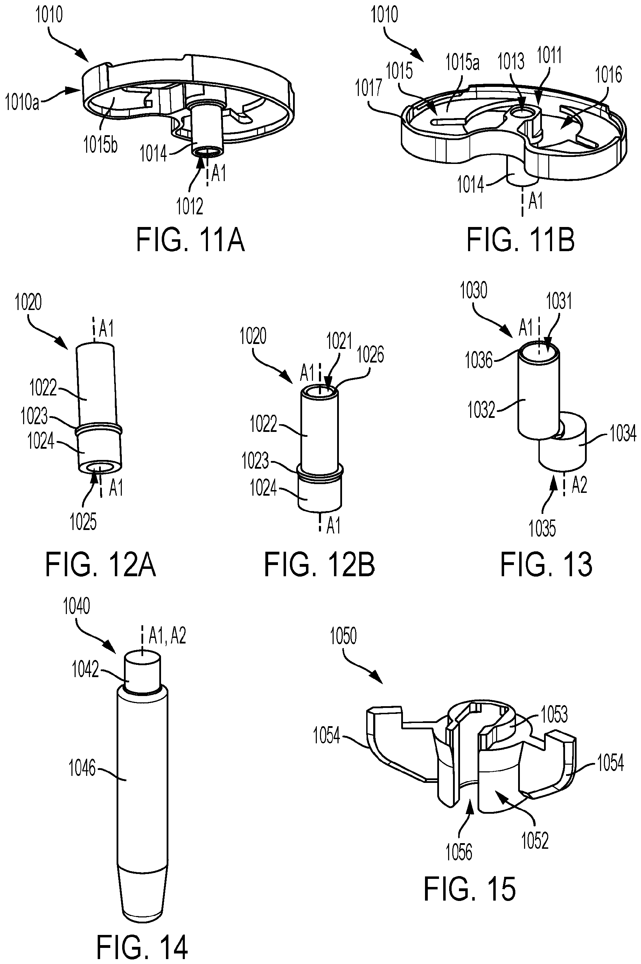

The distal void filler 130 effectively extends the proximal void filler 120 to ensure a bone void in a tibia is filled and baseplate 110 is well supported. In this regard, the outer surface 132a of the proximal boss 132 of the distal void filler 130 is received within bore 112 of the baseplate 110. The distal surface 120c of the proximal void filler 120 engages the proximal surface 130a of the distal void filler 130 such that protrusions 131 of the distal void filler 130 mesh with the grooves 121 of the proximal void filler 120 thereby preventing rotation of the distal void filler 130 relative to proximal void filler 120. The outer surface 130b of the distal void filler 130 may also be press-fit or cemented to the tibia from within the bone void. The head of the locking screw 150 engages the hole 113 of the baseplate 110 while the threaded shaft of screw 150 threadedly engages the bore 133 of the distal void filler 130 thereby securing both the proximal and distal void fillers 120, 130 to baseplate 110, as best shown in FIG. 1C. An IM stem, similar to trial stem 1040 of FIG. 14, may further be connected to distal boss 136 and distal bore 134 for additional support of baseplate 110.

In this configuration, proximal and distal void fillers 120, 130, as well as any IM stem connected thereto, are coaxially aligned with first axis A1. As such, first assembly 100a is a neutral configuration and may be used in circumstances where it is desirable to fill a bone void without the need for a stem offset. It should also be noted that augment 140 may be optional with this configuration depending on the condition of the bone.

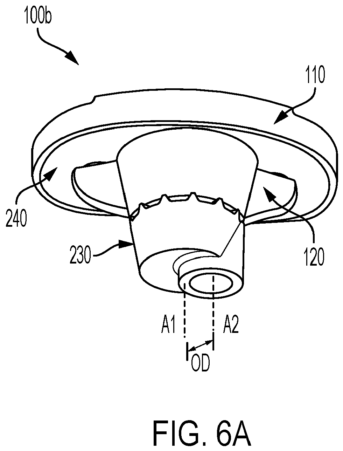

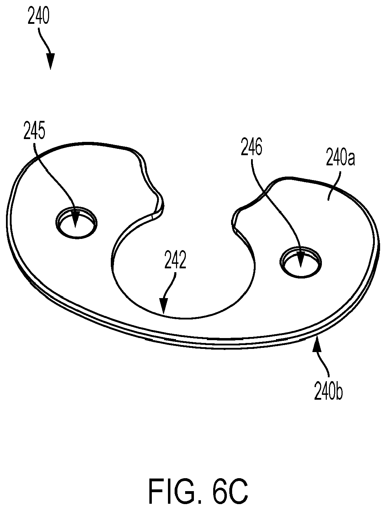

FIGs. 6A-6C depict a second joint replacement assembly 100b. Joint replacement assembly 100b is similar to first assembly 100a in that it includes baseplate 110 and proximal void filler 120. However, assembly 100b includes an offset distal void filler 230 and a full augment spacer 240 in lieu of distal void filler 130 a partial spacer 140. However, it should be understood that partial spacer 140 could be used instead of full spacer 240. This assembly 100b may be utilized when an offset stem is indicated, as explained in more detail below.

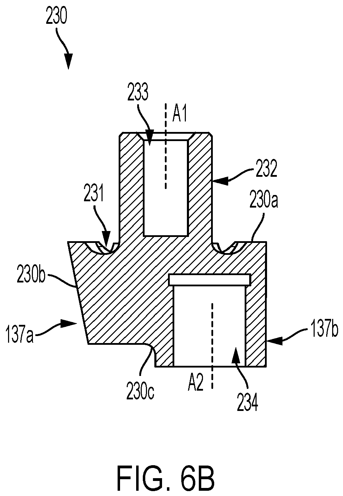

The offset distal void filler or distal member 230 is similar to neutral distal void filler 130 in several respects. Therefore, like elements are accorded like reference numerals to that of void filler 130, but within the 200-series of numbers. For instance, offset distal void filler 230 includes a proximal boss 232, a plurality of teeth or protrusions 231 arrayed about proximal boss 232 for engagement with proximal void filler 120, a proximal threaded bore 233, and a distal bore 234. However, offset distal void filler 130 differs in that the distal bore 234 defines a second axis A2 that is laterally offset by an offset distance OD from a central axis (i.e., first axis A1) passing centrally through the boss 232 and proximal bore 233, as best shown in FIGs. 6A and 6B.

The augment spacer 240 is defined by a proximal surface 240a and a distal surface 240c. The proximal surface 240a and distal surface 240c are generally planar and include first and second holes 245, 246 that are generally cylindrical and disposed opposite one another about a cutout 242. Unlike the augment spacer 140 in joint replacement assembly 100a, the augment spacer 240 spans the entire medial-lateral width of the tibial plateau. It should be understood that augment spacer 240 may be utilized instead of augment spacer 140 in any of the assembly embodiments disclosed herein to the extent such spacer 240 is indicated for a particular patient's malady.

When the joint replacement assembly 100b is fully assembled, the augment spacer 240 is connected to the baseplate 110 such that second and third bosses 114, 116 extend into openings 245, 246 thereby indexing baseplate 110 in the proper orientation. Similar to assembly 100a, proximal and distal void fillers 120, 230 are secured by a screw 150 extending through baseplate 110 and connecting to proximal bore 233 of distal void filler 230 in the same manner shown in FIG. 1C. Depending on the nature of a patient's specific offset, distal void filler 230 can be rotated at predefined increments based on the spacing of teeth 231 so that an intramedullary stem connected to distal bore 234 may be appropriately aligned with an intramedullary canal of a tibia.

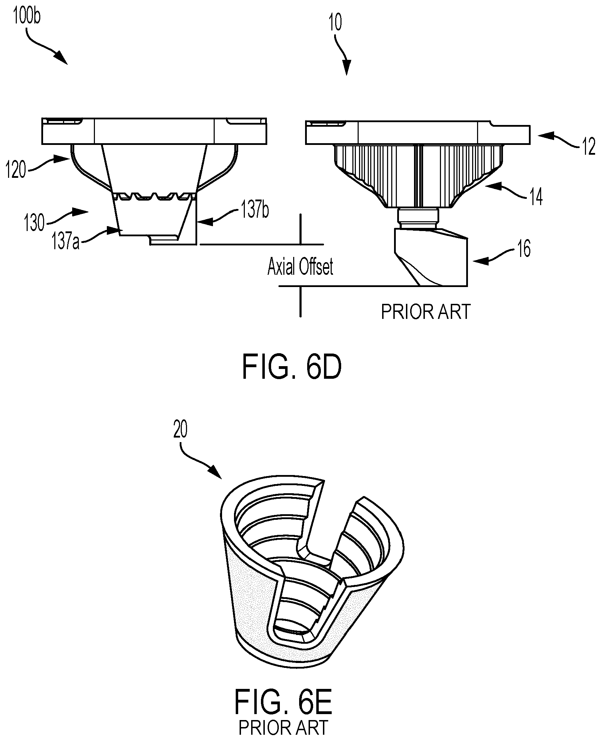

FIG. 6D depicts a side-by-side comparison of second joint replacement assembly 100b and a prior art joint replacement assembly 10. Prior art joint replacement assembly 10 is representative of a common tibial component assembly with a stem offset. In this regard, assembly 10 has a baseplate 12, an integral keel 14, and an offset stem adapter 16 connected to the keel 14. It should first be noted that the prior art assembly 10 does not have interdigitating teeth like that of assembly 100b such that the ability to incrementally and accurately set an offset stem internal-external rotational orientation is lacking. Moreover, as illustrated in FIG. 6D, the offset stem adapter 16 extends farther from baseplate 12 than offset distal void filler 137a and, therefore, extends far deeper into a tibia than assembly 100b. The additional depth is represented by the "axial offset" shown in FIG. 6D. The prior art arrangement creates challenges for filling cavitary defects with cone-type or sleeve-type void fillers as adapter 16 would have to be placed within any of such void fillers, such as the cone-type void filler 20 shown in FIG. 6E. However, as can be seen, void filler 20 is narrower at its distal end which is the point at which adapter 16 would be received within cone void filler 20. Therefore, cone void filler 20 would have to be increased in size to accommodate offset adapter 16. This would be problematic though as it would require more bone removal in a metaphyseal region of bone that is already narrow to begin with which could create a destructively weak thin wall of bone. One possible solution is to position offset adapter 16 deeper within a tibia and beyond a distal end of void filler 20. However, this is also problematic particularly for larger offsets as there may be size restrictions due to the narrowing of the tibia.

Second assembly 100b offers a solution to these issues in that it allows for incremental rotational adjustment of a stem offset. Moreover, the proximal and distal void fillers 120, 130 can satisfactorily fill a bone void while providing a stem offset in an axially compact package. This is facilitated by the modular arrangement of proximal and distal void fillers 120, 130 and also the geometries of distal void filler 130. In this regard, distal void filler 130 has a body portion 137a that is frustoconical in shape and provides a void filling functionality and a lobe portion 137b that is cylindrical and provides an offset stem functionality. Additionally, lobe portion 137b is integrated into a side of the body portion 137a so that lobe portion 137b and body portion 137a occupy the same axial length. In other words, as shown in FIG. 6D, a majority of lobe portion 137b extends between a proximal and distal end of body portion 137a. Thus, rather than being stacked axially, the offset stem and void filling functionalities are arranged side-by-side thereby reducing the amount of axial space occupied in a tibia.

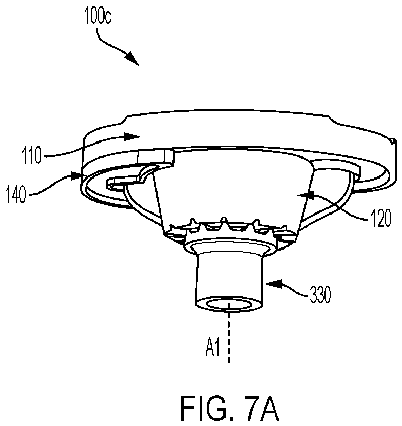

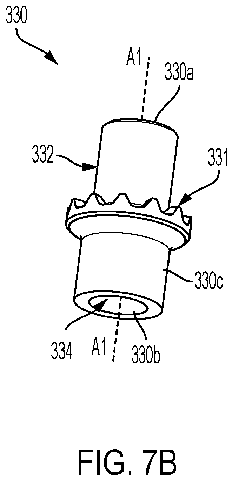

FIGs. 7A-7B depict a joint replacement assembly 100c according to another embodiment of the present disclosure. Assembly 100c is similar to assembly 100a in that it includes baseplate 110, proximal void filler 120, and partial augment 140. However, unlike assembly 100a, assembly 100c includes a distal member that is a neutral stem adapter 330 and does not include the distal void filler 130 of joint replacement assembly 100a. The stem adapter 330 differs from the distal void filler 130 in that it is low profile for situations in which sufficient bone stock is available distal relative to the placement of proximal void filler 120. Thus, adapter 330 differs with regards to its outer surface 330c which is exposed to bone. The outer surface 330c of the stem adapter 330 is generally cylindrical and does not taper from the proximal surface 330a to the distal surface 330b (although it can in some embodiments), while the outer surface 130b of the distal void filler 130 is generally conical and tapers from the proximal surface 130a to the distal surface 130c in order to occupy more space and press-fit into a bone void. Additionally, the cross-sectional dimension of adapter 330 is smaller from teeth 331 to distal surface 330b. As such, the teeth 331 do not extend as far radially outwardly as that of distal void filler 130. Adapter 330 is configured for a neutral arrangement of an intramedullary stem. As such, distal bore is coaxial with first axis A1.

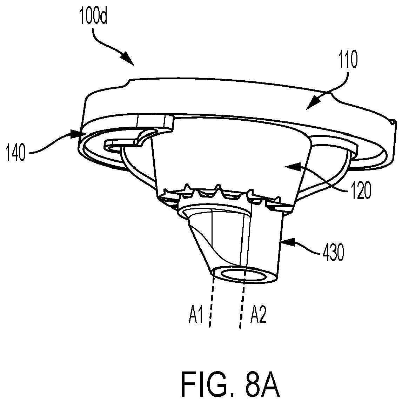

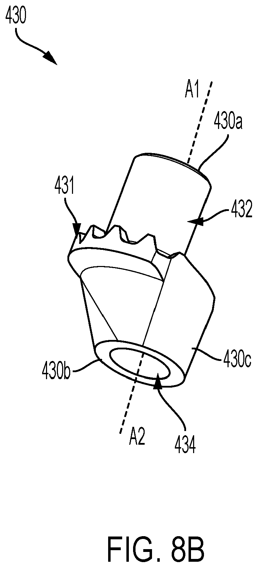

FIGs. 8A-8B depict a joint replacement assembly 100d according to another embodiment of the present disclosure. Assembly 100d is similar to assembly 100a in that it includes baseplate 110, proximal void filler 120, augment spacer 140, and locking screw 150. However, joint replacement assembly 100d includes a distal member that is an offset stem adapter 430 instead of distal void filler 130.

The offset stem adapter 430 is similar to offset void filler 230. Therefore, offset stem adapter 430 is accorded like reference numerals to that of offset distal void filler 230 but within the 400-series of numbers. In that regard, offset stem adapter 430 includes a proximal boss 432 aligned with first axis A1, teeth 431 arrayed about axis A1, and a distal bore 433 aligned with a second axis A2 offset from axis A1 by an offset distance. Such bore 433 is configured to connect to an intramedullary stem. However, offset stem adapter 430 is low-profile in comparison to distal void filler 230, which is intended to fill a bone void that may be the result of disease or otherwise. On the other hand, offset stem adapter 430 may be used in circumstances in which there is sufficient bone stock so that offset distal void filler 230 is not needed but an offset stem arrangement is desired. As such, the footprint or exterior profile of outer surface 430c of the stem adapter 430 is minimized such that it is cylindrical at one side of second axis A2 and tapered at another side of second axis A2. Stated another way, unlike distal void filler 230, distal offset adapter 430 does not have the void filling body portion 137a that is found in distal void filler 230 but does have an offset stem adapter lobe portion, like that of lobe portion 137b. Additionally, the cross-sectional dimension of offset adapter 430 taken perpendicular to axis A2 is smaller from teeth 431 to distal surface 430b than that of distal void filler 230.

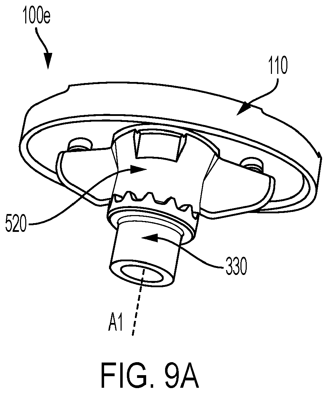

FIGs. 9A-9B depict a joint replacement assembly 100e according to another embodiment of the present disclosure. Assembly 100e is similar to assembly 100a in that it includes baseplate 110 and may or may not include one of the previously discussed augment spacers, such as augments 140 and 240. Also, like assembly 100c, assembly 100e includes the low profile neutral distal adapter 330. But rather than include proximal void filler 120 as the proximal member, assembly 100e includes a lower profile modular keel 520. Thus, assembly 100e may be used in circumstances in which a patient's bone stock is sufficient that it does not warrant the use of the larger footprint void fillers discussed herein.

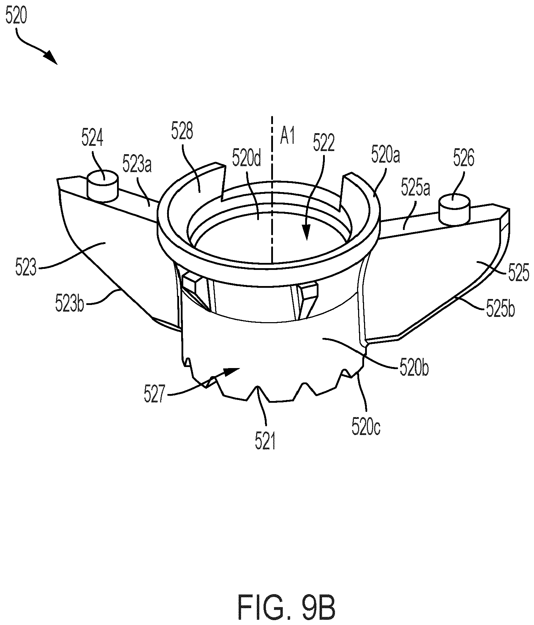

The modular keel 520 is similar to proximal void filler 120 with the exception that modular keel 520 is low profile. Therefore, like elements are accorded like reference numerals to that of proximal void filler 120 but within the 500-series of numbers. Modular keel 520 has a keel body 527 that is defined by a proximal surface 520a, an outer surface 520b, a distal surface 520c, and an inner surface 520d, as best shown in FIG. 9B. The outer surface 520b extends from the proximal surface 520a to the distal surface 520c and is generally cylindrical along at least a portion of a length of keel body 527. In this regard, keel body 527 has a smaller cross-sectional dimension than that of proximal void filler 120. The inner surface 520d defines a bore 522 that is generally cylindrical along at least a portion of a length of body 527. A proximal end of body 527, which includes proximal surface 520a, flares radially outwardly so as to form a collar 528 that extends partially about axis A1. A first fin 523 and a second fin 525 extend in an outward direction from keel body 521, as shown in FIG. 9B. The fins 523, 525 each include protrusions 524, 526, respectively, that are generally cylindrical and extend from proximal surfaces 523a, 525a in a proximal direction. Such protrusions 524, 526 are configured to be received within bosses 114 and 116 in baseplate 110. Distal surface 523b, 525b of the fins 523, 525 are generally planar and are angled to taper down towards the distal surface 520c of the modular keel 520.

The distal surface 520c of the modular keel 520 is defined by the outer circumference of a distal opening of the bore 522. Similar to proximal void filler 120, distal surface 520c includes a plurality of notches or grooves 521 disposed upon the distal surface 520c. The distal surface 520c includes twelve notches or grooves 521 disposed equally along a central axis of the bore 522, i.e., every 30 degrees. However, the distal surface 520c is not limited to this configuration and may include more than twelve grooves 521 or less than twelve grooves 521.

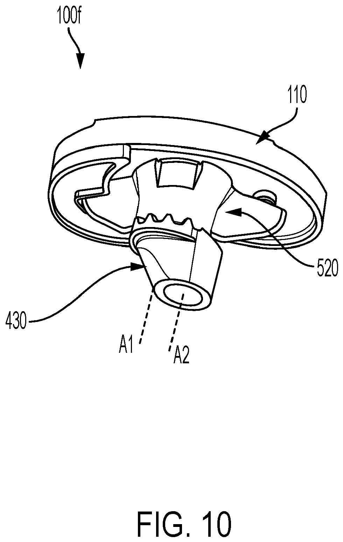

FIG. 10 depicts a joint replacement assembly 100f according to another embodiment of the present disclosure. Joint replacement assembly 100f is another low-profile assembly option for a surgeon similar to that of assembly 100d with the exception that offset stem adapter 430 may be attached to modular keel 520 instead of neutral stem adapter 330. This allows a surgeon to address different surgical scenarios, as discussed further below.

Often when a revision total knee arthroplasty is performed, the particularities of a patient's anatomy do not reveal themselves until the procedure is well underway. For example, the presence and geometries of cavitary and other types of defects that may be caused by disease and/or the removal of an implant may not be known until the previous implant is removed. In that regard, a surgeon may enter a procedure without knowing whether or not a void filler and/or augment spacer are necessary to restructure the joint. Similarly, the patient may need an offset stem or a neutral stem in order to properly align the tibial baseplate on the tibial plateau. Again, which type of stem (offset or neutral) may not be revealed until the procedure is well under way. As such, it is advantageous to provide a flexible system to address most, if not all, situations that may be encountered.

As illustrated by the foregoing embodiments, a kit may be provided that includes each of the previously described devices which may allow a surgeon to assemble any one of the foregoing assemblies (i.e., assemblies 100a-f) depending on the situation at hand. For example, a kit may include tibial baseplate 110, augment spacers 140 and 240, proximal void filler 120, neutral and offset distal void fillers 130 and 230, neutral and offset adapters 330 and 430, and modular keel 570. Such a kit would allow the surgeon to build whichever assembly is necessary to address a particular patient's bone structure as assessed during a surgical procedure.

FIGs. 11A-17 depicts some instrumentation that may be used to prepare a tibia to receive any one of the foregoing joint replacement assemblies 100a-f. Such instrumentation includes a tibial trial 1010, a neutral trial stem extender 1020, an offset stem trial extender 1030, a trial stem 1040, a keel punch 1050, a reamer assembly 1070, and a cutting guide 1090.

FIGs. 11A and 11B depict tibial trial 1010 which generally includes a baseplate portion 1010a and an attachment member 1011. Baseplate portion 1010a includes a plate 1015 with a proximal surface 1015a and opposing distal surface 1015b and a rim 1017 extending about plate 1015 which forms a tray configured to receive a tibial trial insert (not shown). An opening 1016 extends through plate 1015 from the proximal surface 1015a to the distal surface 1015b and is shaped to receive keel punch 1050. Attachment member 1011 is positioned within the opening 1016 and connects to rim 1017. Attachment member 1011 includes a boss 1014 that extends distally relative to plate 1015 and is configured to connect to neutral and offset stem trial extenders 1020, 1030. Additionally, a bore 1013 extends through attachment member 1011 and is coaxial with first axis A1.

FIGs. 12A and 12B depict a neutral trial stem extender 1020 which generally includes a proximal portion or first portion 1022 and a distal portion or second portion 1024. Proximal portion 1022 is cylindrical and includes a proximal opening or bore 1021 extending therein along first axis A1. Distal portion 1024 extends from proximal portion 1022 and is also cylindrical but with a greater outer diameter than proximal portion 1022. Distal portion 1024 also includes distal opening or bore 1025 extending therein which is coaxial with proximal opening 1021 and first axis A1. Proximal opening 1021 is dimensioned to receive boss 1014 of tibial trial 1010, while distal opening 1025 is dimensioned to receive a boss 1042 of trial stem 1046. However, in other embodiments, proximal portion 1022 may be dimensioned to be received within bore 1012 of boss 1014 and/or distal opening 1025 may be dimensioned to be received within an opening (not shown) in an alternative embodiment of trial stem 1040.

FIG. 13 depicts offset stem trial extender 1030 which generally includes a proximal portion or first portion 1032 and a distal portion or second portion 1034. Proximal portion 1032 is cylindrical and includes a proximal opening or bore 1031 extending therein along first axis A1. Distal portion 1034 extends from proximal portion 1032 and is also cylindrical but with a greater outer diameter than proximal portion 1032. Distal portion 1034 also includes distal opening or bore 1035 extending therein and along second axis A2 which is offset from first axis A1. Proximal opening 1031 is dimensioned to receive boss 1014 of tibial trial 1010, while distal opening 1035 is dimensioned to receive boss 1042 of trial stem 1040. However, in other embodiments, proximal portion 1032 may be dimensioned to be received within bore 1012 of boss 1014 and/or distal opening 1035 may be dimensioned to be received within an opening (not shown) in an alternative embodiment of trial stem 1040.

FIG. 14 depicts trial stem which includes a stem portion and a boss extending from stem portion. As illustrated, boss and stem portion may be coaxially aligned with first axis A1 or second axis A2 depending on whether stem is connected to neutral or offset stem trial extender. Trial stem is also representative of an implant stem which can be connected to any one of neutral distal void filler 130, offset distal void filler 230, neutral stem adapter 330, and offset stem adapter 430 for permanent implantation along with any one of assemblies 100a-f.

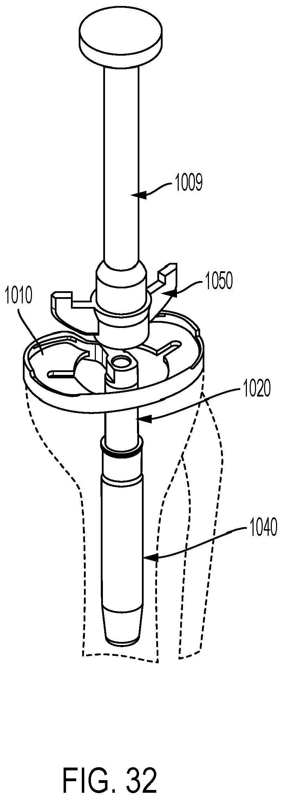

FIG. 15 depicts a keel punch 1050 that includes a body 1052 and fins 1054 extending from body 1052. Body 1052 includes a boss 1053 extending therefrom at a proximal end thereof for connection to an impactor instrument 1009 (see FIG. 32). Fins 1054 extend radially outwardly from body 1052 and are configured to punch through bone to produce elongate slits configured to receive corresponding fins 1054 of proximal void filler 120 or modular keel 520. A slot 1056 extends through body 1052 in a lengthwise direction. Such slot 1056 is configured to slidingly receive attachment member 1011 of baseplate trial 1010.

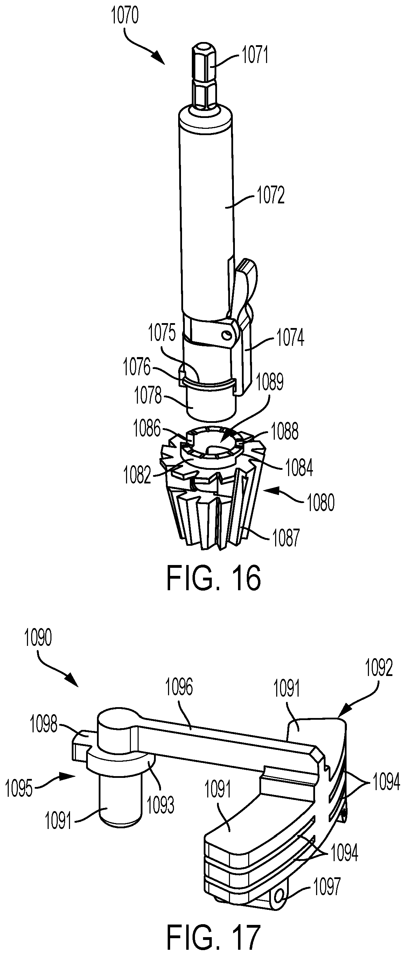

FIG. 16 depicts a reamer assembly 1070 which generally includes a reamer shaft 1072 and reamer head 1080. Reamer shaft 1072 has a proximal engagement member 1071 for engaging a torque applying device, such as an electric drill. A distal end 1078 of reamer shaft 1072 is cylindrical and includes a circumferential rim 1075 that operates as a depth stop when the distal end 1078 is received within an opening 1089 in reamer head 1080. At one side of shaft 1072, a tab 1076 extends radially outwardly therefrom. A spring-loaded latch 1074 extends over rim 1075 so that it can engage reamer head 1080 and secure it to the distal end 1078 of reamer shaft 1072.

Reamer head 1080 is generally frustoconical and includes cutting features 1087 along an exterior thereof. An opening or bore 1089 extends into a proximal end of reamer head 1080. A boss 1082 extends from the proximal end of reamer head 1080 and about opening 1089 such that boss 1082 forms an annulus. A proximal surface of boss 1082 has a plurality of markings 1088 spaced at predetermined intervals that indicate incremental degrees of internal-external rotation relative to a longitudinal axis of reamer head 1080. A recess 1084 extends into boss 1082 radially inwardly and is configured to engage latch 1074 for connection thereto. At an opposite side of boss 1082, a notch 1086 extends in a proximal to distal direction and is configured to receive tab 1076 of shaft 1072 to help ensure rotation of shaft 1072 is duly imparted on reamer head 1080. Notch 1086 also acts as an indexing feature to set a rotational alignment of any tool connected to it, such as cutting guide 1090, for example.

FIG. 17 depicts cutting guide 1090 which generally includes a guide body 1092, an arm 1096, and an attachment member 1095. Guide body 1092 includes a plurality of captured slots 1094 extending therethrough and uncaptured guide surfaces 1091 at a proximal end of guide body 1092. Captured slots 1094 may be configured for augment cuts while guide surfaces 1091 may be configured for clean-up cuts. Thus, captured slots 1094 may be separated by increments associated with different thickness spacer augments, such as augments 140 and 240, for example. Guide body 1092 is connected to one end of arm 1096, while attachment member 1095 is connected to the other end of arm 1096. Attachment member 1095 includes a downwardly extending boss 1091 that is configured to be received within opening 1089 in reamer head 1080. A tab 1098 extends radially outwardly from attachment member 1095 and is configured to be received within notch 1086 of reamer head 1080 to thereby index the rotational alignment of cutting guide 1090 relative to reamer head 1080.

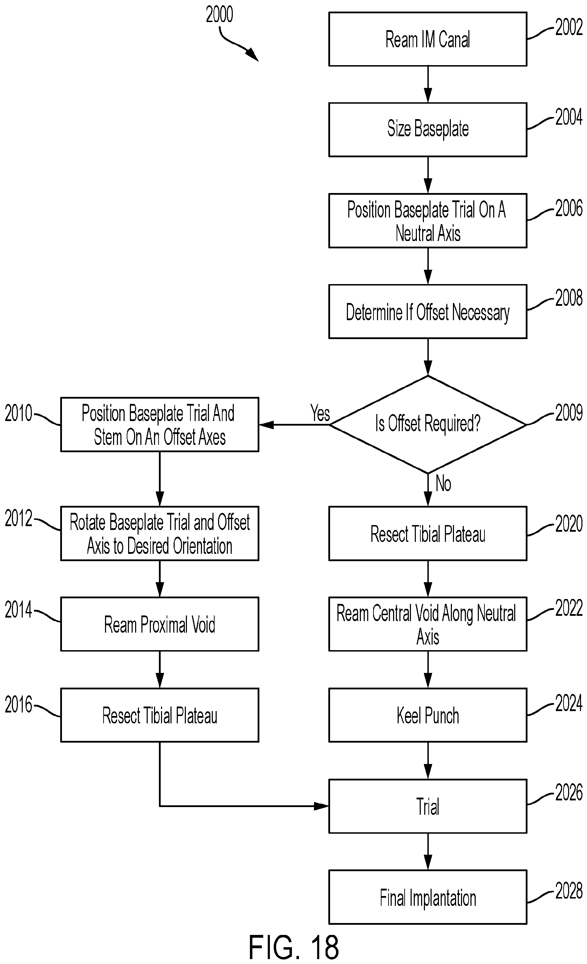

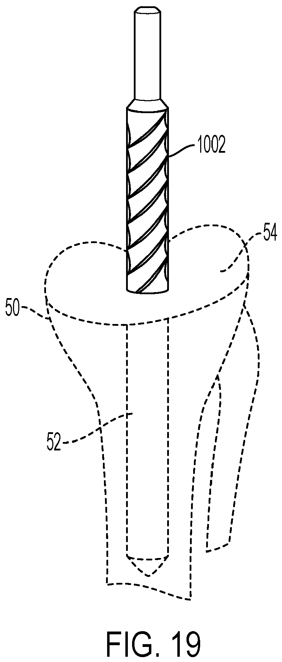

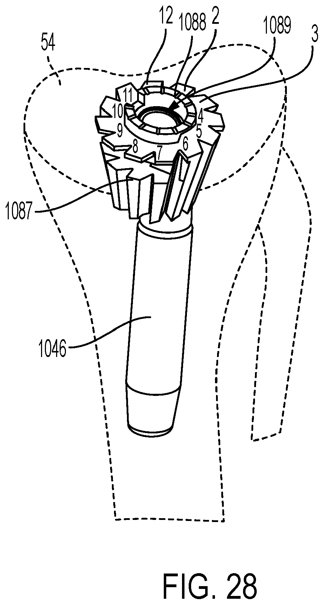

FIG. 18 is a flow chart illustrating a method 2000 of preparing bone for any one of the assemblies 100a-f described herein. Additionally, FIGs. 19-33 depict various operations of the method 2000. It should be understood that the following operations do not have to be performed in the exact order described below and are not necessarily exhaustive of all of the operations that may be performed during a bone preparation procedure. Additionally, it should be understood that the below operations may be handled in a different order or simultaneously.

As shown in FIG. 19, an operation 2002 of the method includes reaming an IM canal 52. In this regard, an intramedullary reamer 1002 is used to ream an intramedullary canal 52 of a tibia 50. The intramedullary canal 52 may be continuously reamed until a desired stem size is attained. The intramedullary reamer 1002 is removed.

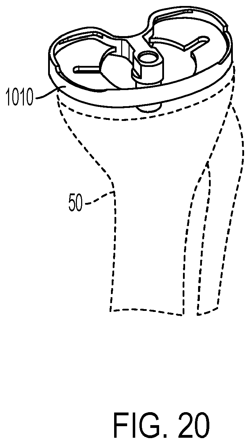

In another operation 2004 of the method, which is depicted in FIG. 20, a tibial baseplate 1010 is sized by placing one or more sizes of tibia baseplate trial 1010 onto a tibial plateau 54. It should be noted that in a revision procedure, the tibial plateau 54 is ordinarily already resected as a result of a previous arthroplasty procedure. Thus, tibial plateau 54 is a resected plateau. However, further resection of the plateau 54 is often performed as a matter of course which may be performed further on in the procedure, as described below. The baseplate trial 1010 is assessed for proper mediolateral (ML) and anteroposterior (AP) fit or coverage of plateau 54. An appropriate size adjustment is made to baseplate trial 1010 as necessary.

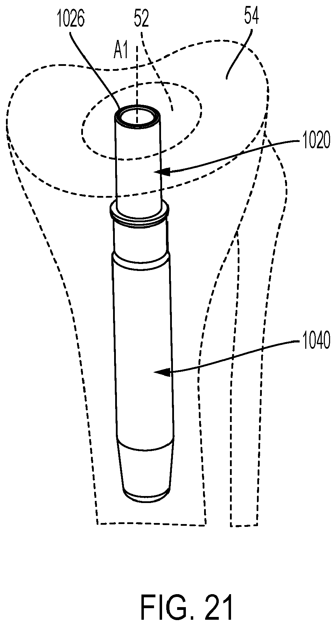

In a further operation 2006 of the method, the baseplate trial 1010 is aligned with a neutral axis (i.e., first axis A1) so that that an operation 2008 of determining whether an offset is necessary can be performed. In this regard, an appropriate stem trial 1040 is connected to a neutral stem trial extender 1020 and is positioned within the intramedullary canal 52, as best shown in FIG. 21. The neutral stem trial extender 1020 is preferably positioned within the intramedullary canal 52 so that a proximal end 1026 is approximately flush with the tibial plateau 54.

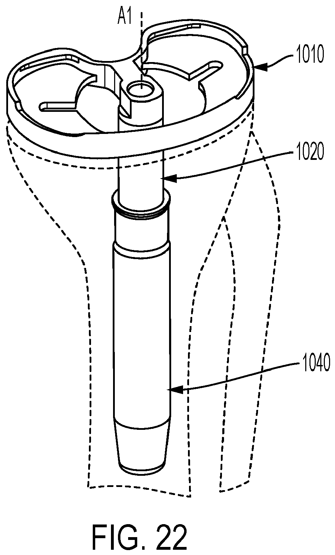

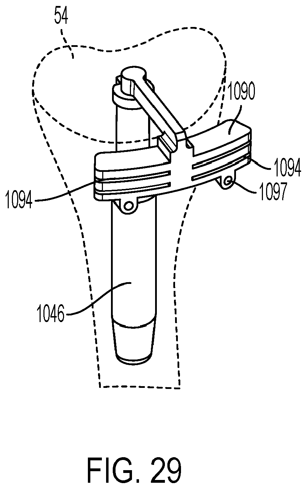

As shown in FIG. 22, once the neutral stem trial extender 1020 is sufficiently positioned, the tibia baseplate trial 1010 is connected to the proximal portion 1022 of the neutral stem trial extender 1020 to reassess ML and AP fit or coverage. If the ML and AP coverage is inadequate, use of an offset stem trial extender 1030 may be necessary to ensure baseplate trial 1010 is properly arranged on tibial plateau 54. As illustrated in the flow chart of FIG. 18, if ML and AP coverage is adequate and therefore no offset is required, an operation 2020 of resecting the tibial plateau 54 is commenced, as shown in FIG. 29 and explained in further detail below.

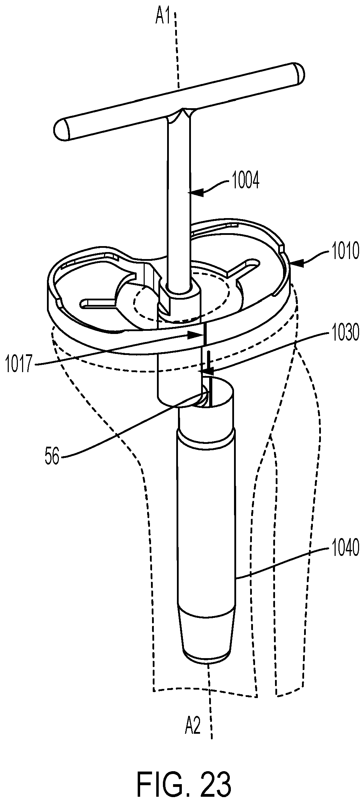

Should the ML and AP coverage be deemed inadequate such that a stem offset 1030 is required, an operation 2010 of positioning baseplate trial 1010 and trial stem 1040 an offset axes A1, A2 is commenced. In this regard, baseplate trial 1010, the neutral stem trial extender 1020, and the stem trial 1040 are removed from the tibia 50. Stem trial 1040 is then connected to offset stem trial extender 1030 and is positioned within the IM canal 52. The offset stem trial extender 1030 may be 2, 4, 6, or 8 mm in length, for example. The appropriately sized baseplate trial 1010 is then connected with a "T" handle 1004 and to offset stem trial extender 1030, as shown in FIG. 23.

Thereafter, an operation 2012 is performed to find the desired rotational orientation of the second axis A2 (i.e., offset axis) about first axis A1 that provides optimal trial baseplate coverage. As such, the "T" handle 1004 is rotated about axis A1 until the baseplate trial 1010 is adequately positioned on the tibial plateau 54, as best shown in FIG. 23. After the baseplate trial 1010 has been adequately positioned, a tibia marking 56 is created to match the orientation of the anteriorly placed baseplate trial marking 1017. The tibia marking 56 can be created using a bovie or a methylene blue pen. The baseplate trial 1010 and "T" handle 1004 are then removed.

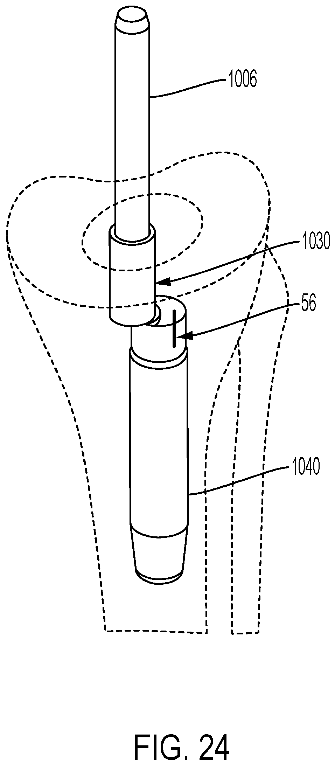

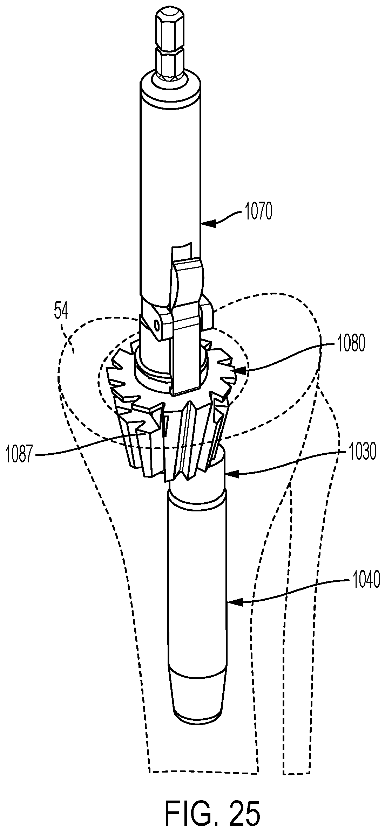

An operation 2014 of reaming a proximal void 2014 may then commence. As such, a reamer guide shaft 1006 is positioned within the offset stem trial extender 1030, as best shown in FIG. 24. An appropriate cone reamer head 1080 and cone reamer drive shaft 1072 are then advanced over the reamer guide shaft 1006 and into the bone, as best shown in FIG. 25. The appropriate depth may be reached once reamer head 1080 contacts distal portion 1034 of offset trial stem extender 1030. Thereafter, the reamer shaft 1072 is disconnected from the reamer head 1080 and removed from the assembly 1070, leaving the reamer head 1080 securely embedded within the bone.

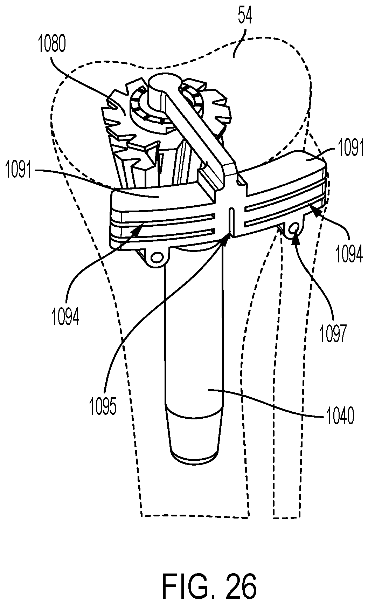

Thereafter, an operation 2016 of resecting the tibial plateau 54 may be performed. In this regard, cutting block or guide 1090 is connected to the reamer head 1080, as best shown in FIG. 26. Such connection is performed by inserting boss 1091 of attachment member 1095 into opening 1089 in reamer head 1080. To properly align the cutting block 1090 and to help ensure proper orientation of anterior-posterior slope, a mid-line marking 1095 disposed on a surface of the cutting block 1090 is aligned with the tibia marking 56. Once properly aligned, the cutting block 1090 is secured to the tibia 50 with fixation pins/drills inserted through pin guide holes 1097. Once secured, clean-up resurfacing cuts are performed to align the tibial plateau 54 with the clean-up cutting surface 1091 of the cutting block 1090. Further cuts may be performed using augment cut slots 1094 for positioning of an augment trials, similar to augments 140 and 240, on a tibia. More specifically, a 5 mm or a 10mm augment cut through associated slots 1094 may be used to match the size of the augment spacer 140, 240 to be implanted into the tibia 50, for example.

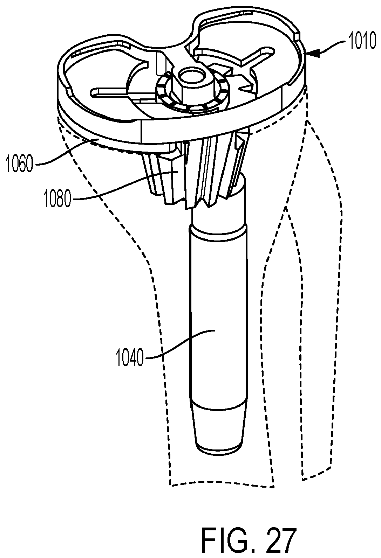

After performing the appropriate cuts, a trialing operation 2026 is performed. In this regard, the cutting block 1090 is disconnected from the cone reamer head 1080 and removed from the tibia 50. The baseplate trial 1010 is connected to the cone reamer head 1080 and positioned on the tibial plateau 54, as best shown in FIG. 27. Augment trials 1060 may be connected to the baseplate trial 1010 if necessary. A trial reduction may then be performed to evaluate passive joint function and stability.

Once the trial reduction is deemed adequate, a final implantation operation 2028 may be performed. As such, the baseplate trial 1010 may be disconnected from the cone reamer head 1080. Prior to removing the cone reamer head 1080, the offset position of the cone reamer head 1080 is recorded, with respect to the offset stem trial extender 1030, based upon the cone reamer head markings 1088. The offset position recorded may be used for implanting one of joint replacement assemblies 100b, 100d, and 100f. The recorded offset position may be used to set the rotational orientation of distal offset void filler 230 or distal offset adapter 430 by matching up the appropriate teeth 231, 431, and notches 121, 521 with the recorded offset position.

Returning now to the scenario in which no offset is required and after the baseplate 1010 has been sized, an operation 2020 of resecting the tibial plateau 54 may commence. At this point, the stem trial and neutral stem extender 1020, 1040 are positioned within the IM canal 52. The cutting block 1090 is assembled and connected to the neutral stem trial extender 1020, as shown in FIG. 29. Again, the cutting block 1090 is secured to the tibia 50 with fixation pins/drills inserted through pin guide holes 1097. Once secured, clean-up resurfacing cuts are performed to align the tibial plateau 54 with the clean-up cutting surface 1091 of the cutting block 1090. Further cuts may be performed in the augment cut slots 1094 for positioning of an augment trial 1060 within the tibia 50. Captured slots 1094 may be spaced from each other in 5 mm increments, for example, such that at least a portion of tibial plateau 54 may be reduced in corresponding increments to match the size of the augment trial 1060 to be trialed. After performing the appropriate cuts, the cutting block 1090 is disconnected from the neutral appropriate stem trial extender 1020 and removed from the tibia 50.

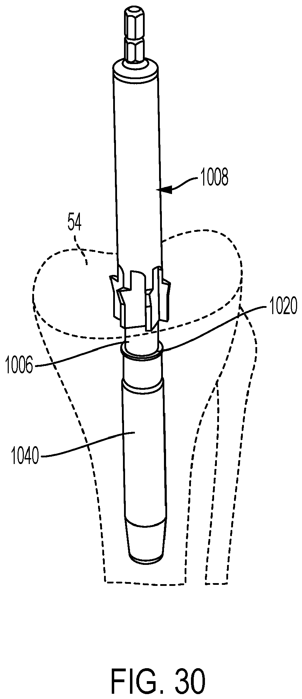

Thereafter, an operation 2022 of reaming a central void along a neutral axis is performed. In this regard, reamer guide shaft 1006 is positioned within the neutral stem trial extender 1020. Thereafter, a keel boss reamer 1008 is positioned over the reamer guide shaft 1006, as best shown in FIG. 30. The keel boss reamer 1008 is then advanced along guide shaft 1006 and into the bone to ream the tibia 50 After reaming, the keel boss reamer 1008 and the reamer guide shaft 1006 are removed from the tibia 50.

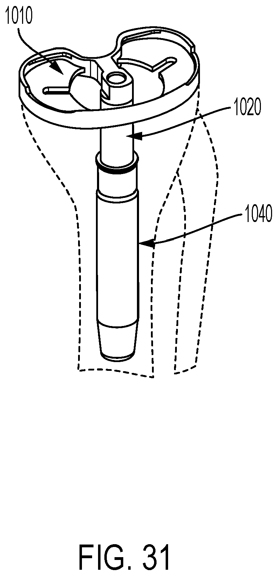

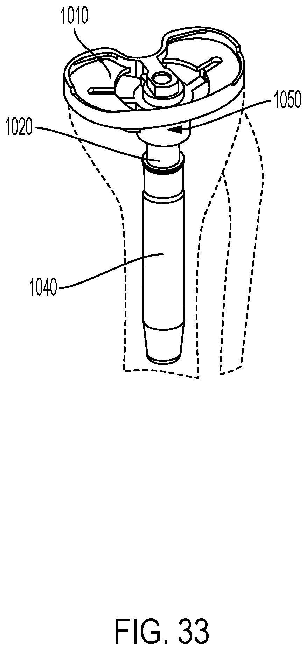

Once removed, a keel punch operation 2024 is performed. In this regard, baseplate trial 1010 is connected to neutral stem trial extender 1020, as best shown in FIG. 31. Thereafter, keel punch 1050 is attached to impactor tool 1009 and advanced over attachment member 1011 of baseplate trial 1010 and through the baseplate trial 1010, as best shown in FIG. 32. The keel trial/punch 1050 is impacted into the tibia 50 until it is fully seated onto attachment member 1011 of baseplate trial 1010. At this point, impactor 1009 is disconnected from the keel trial 1050 so that the keel trial 1050 remains within the bone as a trial, as shown in FIG. 33.

After punching the bone with keel punch/trial 1050, a trialing operation 2026 is performed. In this regard, a trial reduction may then be performed to evaluate passive joint function and stability.

Once the trial reduction is deemed adequate, final implantation 2028 may be performed. As such, any one of the neutral assemblies, such as assemblies 100a, 100c, and 100e may be implanted into the prepared bone.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. A joint replacement assembly (100a, 100b, 100c, 100d, 100e), comprising:
a baseplate (110) having an articulating side and a bone facing side;
a proximal member (120, 520) having a proximal end (120a, 520a) and a distal end (120c, 520c), the proximal end being connectable to the bone facing side of the baseplate, the distal end having a plurality of grooves (121, 521) arranged about a first axis (A1) extending through the proximal member in a lengthwise direction; and
a distal member (130, 230, 330, 430) having a proximal end (130a, 230a, 330a, 430a) and a distal end (130c, 230c, 330c, 430c), the proximal end being connectable to the distal end of the proximal member and having a plurality of protrusions (131, 231, 331, 431) configured to engage with the plurality of grooves and prevent rotation of the distal member about the first axis relative to the proximal member.

2. The joint replacement assembly of claim 1, wherein the plurality of grooves are arranged in 30 degree increments about the first axis.

3. The joint replacement assembly of any one of claims 1-2, wherein the distal end of the distal member includes a distal bore (134, 234, 334, 434) configured to engage an intramedullary stem.

4. The joint replacement assembly of claim 3, wherein the distal bore (134, 334) is coaxial with the first axis of the proximal member.

5. The joint replacement assembly of claim 3, wherein the distal bore (234, 434) is centered about an axis (A2) laterally offset from the central axis of the proximal member.

6. The joint replacement assembly of any one of claims 3-5, wherein the proximal member includes a proximal bore (122, 522) coaxial with the first axis.

7. The joint replacement assembly of any one of claims 1-6, wherein the baseplate, proximal member, and distal member are each formed separately from each other.

8. The joint replacement assembly of any one of claims 1-7, wherein a distal surface (110b) of the baseplate includes a hole (114, 116, 118) extending through the baseplate.

9. The joint replacement assembly of claim 8, wherein a proximal surface (110a) of the proximal member includes a boss (123, 524, 526) configured to engage with the hole of the baseplate to prevent rotation of the proximal member relative to the baseplate.

10. The joint replacement assembly of any one of claims 1-9, wherein the distal member includes a proximally extending boss (132, 232, 332, 432) and the baseplate includes a distally extending boss (111), the proximally extending boss being extendable through the proximal member and receivable within the distally extending boss of the baseplate.

11. The joint replacement assembly of claim 10, wherein the proximally extending boss of the distal member is threaded such that a fastener (150) extending through the baseplate and engaging the proximally extending boss secures the baseplate, proximal member, and distal member together.

12. The joint replacement assembly of any one of claims 1-11, wherein the proximal member is a proximal void filler having a frustoconical body having an outer surface (120b, 520b) tapering inwardly from the proximal end to the distal end thereof.

13. The joint replacement assembly of any one of claims 1-11, wherein the distal member is a distal void filler having a frustoconical body having an outer surface (130b, 230b, 330b, 430b) tapering inwardly from the proximal end to the distal end thereof.

14. The joint replacement assembly of claim 13, wherein the distal member further includes a lobe portion (137b) integral with the frustoconical body, the lobe portion extending along at least a portion of a length of the frustoconical body and having an axial bore (234, 434) extending into the lobe, the axial bore defining a second axis (A2) offset from the first axis by an offset distance (OD).

15. The joint replacement assembly of claim 14, wherein the lobe portion and second axis are positionable at a plurality of rotational positions about the first axis.
